## Europäisches Patentamt
## European Patent Office
## Office européen des brevets

(19)

(11) Veröffentlichungsnummer: **0 014 680**
**B1**

(12) ## EUROPÄISCHE PATENTSCHRIFT

(45) Veröffentlichungstag der Patentschrift:
**02.03.83**

(51) Int. Cl.³: **C 07 C 143/55,** C 07 C 139/00, C 07 C 139/14

(21) Anmeldenummer: **80810028.3**

(22) Anmeldetag: **28.01.80**

(54) Verfahren zur Herstellung des Magnesiumsalzes von 3-Nitronaphthalin-1,5-disulfonsäure (Nitro-Armstrong-Säure).

(30) Priorität: **02.02.79 CH 1045/79**

(43) Veröffentlichungstag der Anmeldung:
**20.08.80 Patentblatt 80/17**

(45) Bekanntmachung des Hinweises auf die Patenterteilung:
**02.03.83 Patentblatt 83/9**

(84) Benannte Vertragsstaaten:
**BE CH DE FR GB**

(56) Entgegenhaltungen:
**FR-A-2 413 367**
**US-A-1 756 537**
**US-A-1 836 204**
**US-A-2 191 820**
**US-A-2 875 242**
**CHEMICAL ABSTRACTS, Vol. 60, Nr. 6, 16. März 1964, Spalte 6802e—f Columbus, Ohio, U.S.A. & SU-A-154 261 (A. A. VIGASIN et al.)**

(73) Patentinhaber: **CIBA-GEIGY AG, Patentabteilung Postfach, CH-4002 Basel (CH)**
Patentinhaber: **BAYER AG, Zentralbereich Patente, Marken und Lizenzen, D-5090 Leverkusen 1, Bayerwerk (DE)**

(72) Erfinder: **Albrecht, Bernhard, Im Laig 173, CH-4463 Buus (CH)**
Erfinder: **Frey, Hans, Hauptstrasse 18, CH-4126 Bettingen (CH)**
Erfinder: **Habermacher, Vinzenz, Hammerstrasse 42, CH-4058 Basel (CH)**
Erfinder: **Behre, Horst, Dr., Im Hellsiefen 4, D-5068 Odenthal (DE)**
Erfinder: **Kienitz, Lutz, Dr., Forststrasse 2, D-5060 Bergisch Gladbach 1 (DE)**
Erfinder: **Schenk, Wolfgang, Dr., Odenthaler Strasse 62/64, D-5090 Leverkusen (DE)**
Erfinder: **Steffan, Guido, Dr., Im Herzogenfeld 52, D-5068 Odenthal (DE)**
Erfinder: **Vogel, Axel, Dr., In der Hildscheid 5, D-5068 Odenthal (DE)**

## Verfahren zur Herstellung des Magnesiumsalzes von 3-Nitronaphthalin-1,5-disulfonsäure (Nitro-Armstrong-Säure)

Die vorliegende Erfindung betrifft ein Verfahren zur Herstellung des Magnesiumsalzes von 3-Nitronaphthalin-1,5-disulfonsäure (Nitro-Armstrong-Säure, im folgenden auch als Nitro-AS bezeichnet) durch Nitrierung eines Produktgemisches, das durch Umsetzung von Naphthalin mit Schwefeltrioxid in einem inerten organischen Lösungsmittel erhalten wurde, und Versetzen des mit Wasser verdünnten Nitriergemisches mit einer Magnesiumionen abgebenden Verbindung.

Aus dem US-Patent 2 191 820 ist bekannt, daß man Nitro-AS herstellen kann, indem man zunächst reine Naphthalin-1,5-disulfonsäure und 60%iges Oleum unterhalb von 40°C in Schwefelsäuremonohydrat (100%iges $H_2SO_4$) einträgt. Die Mischung wird dann unterhalb 30°C langsam mit einer wasserfreien Mischung aus Schwefelsäure und Salpetersäure ($HNO_3$-Gehalt 40%) versetzt und 6 bis 7 Stunden bei 30 bis 35°C nachgerührt. Anschließend wird das Nitriergemisch mit Wasser verdünnt und die Nitro-AS nach Zugabe von Magnesiumoxid als Magnesiumsalz isoliert.

Dieses Verfahren bedingt die aufwendige Isolierung von reiner Naphthalin-1,5-disulfonsäure.

Es wurde nun ein Verfahren zur Herstellung des technisch reinen Magnesiumsalzes von 3-Nitronaphthalin-1,5-disulfonsäure durch Sulfonierung von Naphthalin, Nitrierung des Sulfonierungsproduktes und Abtrennung des reinen Magnesiumsalzes von 3-Nitronaphthalin-1,5-disulfonsäure gefunden, das dadurch gekennzeichnet ist, daß man

a) Naphthalin mit flüssigem $SO_3$ in Gegenwart eines reaktionsinerten organischen Lösungsmittels bei Temperaturen im Bereich −40 bis +20°C sulfoniert, wobei das Verhältnis von zugegebenem $SO_3$ zu zugegebenem Naphthalin während der gesamten Zugabe im Bereich von 2,5 bis 5 Mol $SO_3$ pro Mol Naphthalin liegt,

b) dem erhaltenen Reaktionsgemisch eine Mischung, bestehend aus in etwa gleichen Teilen 100%iger Schwefelsäure und ausreagierter roher Nitriermasse aus der Stufe b) zusetzt, wodurch ein Zweiphasen-System resultiert, von dem die obere organische Lösungsmittel-Phase dekantiert und der Rest des verbliebenen organischen Lösungsmittels aus dem AS-AN-Säurebrei destillativ, vorteilhaft durch Flashverdampfung oder gewünschtenfalls bei der nachfolgenden Nitrierung zur gleichzeitigen Abführung der Nitrierungswärme, oder erst nach erfolgter Nitrierung entfernt wird, wonach man zum Rückstand die zur Nitrierung erforderliche Salpetersäuremenge zweckmäßig im Gemisch mit Schwefelsäure und Schwefeltrioxid bei einer Temperatur zwischen 10 und 60°C zusetzt und bei dieser Temperatur während 1 bis 6 Stunden nitriert,

c) danach zunächst Wasser und dann in Abwesenheit des inerten organischen Lösungsmittels bei 90 bis 120°C so viel einer Magnesiumionen abgebenden Verbindung zugibt, daß pro Mol 3-Nitronaphthalin-1,5-disulfonsäure 1,1 bis 1,3 Mol Magnesiumionen zugegeben werden und eine Schwefelsäurekonzentration im Bereich von 40 bis 60 Gew.-% (bezogen auf die Schwefelsäure und Wasser) eingehalten wird, und

d) das ausgefallene Magnesiumsalz der 3-Nitronaphthalin-1,5-disulfonsäure bei einer Temperatur im Bereich von 20 bis 70°C abtrennt und anschließend gegebenenfalls mit maximal 60gew.-%iger Schwefelsäure und/oder Wasser wäscht.

Im erfindungsgemäßen Verfahren werden Naphthalin und $SO_3$, vorzugsweise jeweils getrennt in einem inerten organischen Lösungsmittel, vorzugsweise in einem aliphatischen Chlorkohlenwasserstoff mit 1 bis 3 C-Atomen, insbesondere Dichlormethan, gelöst und beide Lösungen bei ca. −10 bis −5°C vereinigt, wobei während der gesamten Zugabe ein Molverhältnis von zugegebenem $SO_3$ zu zugegebenem Naphthalin im Bereich von 2,5 bis 3,6, vorzugsweise im Bereich von 2,8 bis 3,2, eingehalten wird. Dabei bildet sich ein Sulfonierungsprodukt von Naphthalin, welches sich in fester Form abscheidet und $H_2SO_4$ und gegebenenfalls $SO_3$ enthält. Das Sulfonierungsprodukt liegt als Suspension in dem verwendeten Lösungsmittel vor.

Dieses Sulfonierungsprodukt des Naphthalins, im folgenden auch als Armstrongsäureanhydrid oder AS-AN bezeichnet, ist ein Gemenge oligomerer Anhydride von Naphthalin-di- und -tri-sulfonsäuren mit $H_2SO_4$ und gegebenenfalls $SO_3$. Wenn man die im AS-AN vorhandenen sulfonierten Naphthalineinheiten formal betrachtet als Naphthalinsulfonsäuren und die Schwefelsäure als $SO_3$ auffaßt, so kann das AS-AN-Gemisch beispielsweise 69 bis 88 Gew.-% Naphthalin-di- und -tri-sulfonsäuren und 31 bis 12 Gew.-% $SO_3$ enthalten und enthält im allgemeinen, bezogen auf die Gesamtmenge an Naphthalinsulfonsäuren, über 65 Gew.-% Naphthalin-1,5-disulfonsäure.

Erfindungsgemäß wird bevorzugt AS-AN eingesetzt, das, formal betrachtet, 70 bis 82 Gew.-% Naphthalin-1,5-disulfonsäure, bezogen auf die Gesamtmenge an Naphthalinsulfonsäuren, enthält, wie z. B. ein Gemisch der Zusammensetzung:

75 bis 80 Gew.-% Naphthalin-1,5-disulfonsäure,
0 bis 3 Gew.-% Naphthalin-1,3-disulfonsäure,
5 bis 15 Gew.-% Naphthalin-1,6-disulfonsäure,
2 bis 5 Gew.-% Naphthalin-1,7-disulfonsäure,

0 014 680

0 bis 5 Gew.-% Naphthalin-1,3,5-trisulfonsäure,
0 bis 5 Gew.-% Naphthalin-1,3,6-trisulfonsäure,
0 bis 1 Gew.-% Naphthalin-1,3,7-trisulfonsäure,
0 bis 1 Gew.-% Naphthalin-1-sulfonsäure,

jeweils bezogen auf die Summe aller Naphthalinderivate und gerechnet als freie Sulfonsäure.

Darüber hinaus kann das AS-AN in sehr geringen Mengen weitere, nicht näher identifizierte Dinaphthylsulfone und Dinaphthylsulfonsulfonsäuren enthalten.

Der besondere Vorteil des neuen Verfahrens ist, daß man mit dem rohen Reaktionsgemisch und ohne Isolierung des reinen Sulfonierungsproduktes direkt weiter arbeiten kann.

Die Aufarbeitung des AS-AN zur Trockensubstanz und deren Lagerung und Dosierung erweist sich nämlich als sehr aufwendig. Die Handhabung wie auch das Filtrieren, Trocknen, Lagerung in Silos und Dosieren des AS-AN erfordern kostspielige Spezialapparate, da es stark hygroskopisch ist und außerdem z. B. bei mechanischer Beanspruchung und Temperaturen oberhalb 30°C, leicht zu einer gummiartigen, praktisch nicht mehr weiterverarbeitbaren Masse führt. Die notwendige Niedertemperaturtrocknung würde zudem viel Energie erfordern.

Zur Umgehung dieser Feststoffhandhabung wäre es naheliegend für die nachfolgende Nitrierung direkt das Sulfonierungsgemisch zu verwenden. Dies scheitert jedoch, weil mit zu geringer Schwefelsäuremenge ebenfalls eine gummiartige Masse resultiert; die Verwendung geeigneter Schwefelsäuremengen von mehr als 15 Mol/Mol AS-AN ist großtechnisch aber unwirtschaftlich und ökologisch nicht mehr tragbar.

Es wurde nun gefunden, daß durch Rückführung eines Teils der rohen Nitriermasse aus der Stufe b) überraschenderweise die sonst in konventionellen Verfahren erforderliche Schwefelsäuremenge auf bis zu 20% reduziert werden kann, und daß das AS-AN zudem in einem für die Weiterverarbeitung gut geeigneten pumpbaren Brei erhalten wird.

Das in Stufe b) zur Phasentrennung nach der Sulfonierungsreaktion einzusetzende Säuregemisch aus konzentrierter Schwefelsäure und ausreagierter Nitriermasse läßt sich variieren, doch soll die Gesamtmenge des Gemisches vorteilhaft aus mindestens 6 Gewichtsteilen, und die beiden einzelnen Komponenten aus mindestens je 3 Gewichtsteilen pro 1 Teil in die Sulfonierungsreaktion eingesetztem Naphthalin bestehen. Werden die beiden Komponenten nicht als Säuregemisch, sondern als Einzelkomponenten in beliebiger Reihenfolge der Sulfonierungsmasse zugesetzt, dann entsteht eine unrührbare Masse. Die Verwendung des Säuregemisches ist somit von entscheidender Bedeutung.

Hat sich nach Zusatz des Gemisches aus konzentrierter Schwefelsäure und rohes Nitrierungsreaktionsgemisch zum AS-AN-Gemisch die organische Phase abgetrennt, so wird diese abdekantiert oder abgepumpt und der Rest des organischen Lösungsmittels aus dem AS-AN-Brei destillativ entfernt. Das im Reaktor verbleibende Reaktionsgemisch wird zur Nitrierung mit der erforderlichen Salpetersäuremenge zweckmäßig im Gemisch mit Schwefelsäure und Schwefeltrioxid bei einer Temperatur zwischen 10 und 60°C versetzt und bei dieser Temperatur während 1 bis 6 Stunden nitriert.

Eine besonders bevorzugte Ausführungsform der Stufe b) des neuen Verfahrens besteht darin, daß man den nach der Phasentrennung erhaltenen AS-AN-Brei und die Mischsäure entweder taktweise hintereinander oder simultan in ein vorgelegtes rohes Nitriergemisch dosiert und anschließend wie vorher beschrieben nitriert.

Bei dieser Verfahrensvariante wird das rohe Nitriergemisch in 3 Teile aufgeteilt. Ein Teil wird zum Produkt 3-Nitronaphthalin-1,5-disulfonsäure aufgearbeitet, indem man es in Wasser bei 80 bis 100°C löst und als Magnesiumsalz isoliert. Der zweite Teil wird mit 100%iger Schwefelsäure im Verhältnis 1 : 1 gemischt und zum Zwecke der Phasentrennung dem Sulfonierungsgemisch zugegeben. Der dritte Teil dient als Vorlage für die genannte bevorzugte Ausführungsform einer nächsten Nitrierungsreaktion.

Die vorher genannte Entfernung der letzten Reste des Lösungsmittels aus dem Reaktionssystem, die nach der Dekantierung noch verbleiben, stellt kein grundsätzliches Problem dar. Deshalb kann die Entfernung dieser Reste fakultativ entweder vor der Nitrierungsreaktion durch eine sogenannte Flashverdampfung (Schnellverdampfung) unter Anwendung von Vakuum oder aber erst während der Nitrierung durch Destillation unter Ausnützung der Nitrierungswärme erfolgen, wodurch externe Kühlsysteme zur Abkühlung der Reaktion eingespart werden können.

Die Nitrierung in Stufe b) des erfindungsgemäßen Verfahrens kann sogar in Anwesenheit des im Sulfonierungsprodukt vorhandenen Lösungsmittels durchgeführt werden, vorzugsweise jedoch in Abwesenheit.

Die Trennoperation des Zweiphasen-flüssig-Gemisches nach der Sulfonierungsreaktion erfolgt vorzugsweise bei Raumtemperatur, das Abtrennen des Restlösungsmittels aus der Säurephase vorteilhafterweise durch sogenannte Flashverdampfung bei ca. 10 bis 30°C und 0,027 bis 0,133 bar. Das durch Dekantation und Flash-Destillation rückgewonnene Lösungsmittel kann direkt wieder in der Sulfonierungsreaktion verwendet werden. Es entstehen dann praktisch keine Lösungsmittelverluste, was das Verfahren außerordentlich ökologisch gestaltet.

Als reaktionsinerte, organische Lösungsmittel für das vorliegende Verfahren kommen vor allem Chlorkohlenwasserstoffe, wie z. B. Dichlormethan, Chloroform, Tetrachlormethan, 1,2-Dichloräthan, in

3

Betracht, wegen Energieersparnisüberlegung aber insbesondere leicht siedende Chlorkohlenwasserstoffe. Bevorzugt ist Dichlormethan.

Das für die Nitrierung bevorzugte simultane Dosieren des AS-AN-Breis und der Mischsäure zu einer Restmenge Nitriermasse ermöglicht im technischen Maßstab von Anbeginn an das Abführen der hohen Nitrierwärme und das Einhalten der erfindungsgemäß geforderten Temperaturgrenzen von 10 bis 60°C, vorzugsweise von 35 bis 45°C. Anderenfalls wäre ein externer besonderer Kühlkreislauf erforderlich. Diese Arbeitsweise ist auch vorteilhaft, weil sich Dosierschwankungen nicht nachteilig auswirken und die Prozeß-Sicherheit selbst bei massiven Dosierfehlern aber auch bei anderen Störungen, wie z. B. Ausfall der Kühlung, stets gewährleistet ist.

Die Dosiergeschwindigkeit wird vorzugsweise so gewählt, daß sie etwa Nitriergeschwindigkeit entspricht. Vorzugsweise liegt die Dosierzeit im Bereich zwischen 1 und 3 Stunden. Nach beendeter Dosierung läßt man das Reaktionsgemisch im Verlauf von 0,5 bis 3 Stunden bei 10 bis 60°C ausreagieren. Vorzugsweise beträgt die Nachreaktionszeit 1 bis 2 Stunden bei 35 bis 45°C.

Zur Nitrierung verwendet man vorteilhaft 1 bis 1,5 Mol Salpetersäure conc. in Form von Mischsäure pro Mol eingesetztem Naphthalin. Zweckmäßig besteht die Mischsäure des weiteren aus conc. Schwefelsäure, die noch 0,8 bis 1,2 Mol $SO_3$ pro Mol Salpetersäure enthält.

Die Stufe c) des vorliegenden Verfahrens erfüllt insbesondere eine partielle Reinigungsfunktion und bringt verfahrenstechnische Vorteile, indem das Produkt in einer leicht zu isolierenden Kristallform erhalten wird.

Den größten Anteil an unerwünschtem Nebenprodukt stellt die 3-Nitronaphthalin-1,6-disulfonsäure dar. Diese Verbindung bildet wie die Nitro-Armstrongsäure (3-Nitronaphthalin-1,5-disulfonsäure) ein schwer lösliches Magnesiumsalz.

Bei der Weiterverarbeitung der Nitro-AS zu C-Säure ist es jedoch erforderlich, dieses Nebenprodukt vorher zu entfernen, da die C-Säure sehr gut löslich ist und normalerweise durch Eindampfen der Reaktionslösung gewonnen wird.

Überraschenderweise können durch Kristallisation bei 90—120°C und Filtration des Nitrierungsgemisches in Form der Magnesiumsalze bei 60°C die Nebenprodukte einschließlich der 3-Nitronaphthalin-1,6-disulfonsäure weitgehend in Lösung gehalten und mit der Mutterlauge abgetrennt werden und als Filterrückstand bleibt praktisch reines Nitro-Armstrongsäure-dimagnesiumsalz zurück.

Dies ist von besonderer Bedeutung, denn die Isolierung des Magnesiumsalzes der Nitro-AS durch Fällung, Filtration und Wäsche nach den literaturbekannten Verfahren bereitet erhebliche Schwierigkeiten, erfordert einen hohen Aufwand und ist in genügend reiner Form kaum möglich, weil damit ein sehr feiner Kristallbrei als thixotrope Phase erhalten wird, der sich außerordentlich schlecht filtrieren und waschen läßt, weshalb der Filterkuchen eine hohe Restfeuchte aufweist und stark verunreinigt ist. Als technisch gangbarer Weg hat sich bisher lediglich die mühsame Wäsche des Filterkuchens mit wäßriger Natriumchloridlösung bewährt, was aber eine Verunreinigung des Magnesiumsalzes der Nitro-AS mit Natriumchlorid zur Folge hat.

Die Filtration der Kristalle des Mg-Salzes der Nitro-As stellt ein echtes Ingenieurproblem und zugleich ein Ökologieproblem dar, da große Mengen verdünnter Schwefelsäure und anschließend Waschwasser zum Nachwaschen benötigt werden.

Überraschend ist deshalb, daß das Nitro-AS-Mg-Salz durch Kristallisation bei höheren Temperaturen gemäß Stufe c)

a)  in Form großer, gut filtrierbarer Kristallkornverteilungen erhalten und zudem
b)  die Ausbeute an reinem Nitro-AS-Mg-Salz noch erhöht wird.

Das Reaktionsgemisch aus der Nitrierung von 1,5-Naphthalindisulfonsäure enthält Schwefelsäure aus der Mischsäurenitrierung. Diese wird verdünnt und dient bei der Auskristallisation als Reaktionsmedium.

Aufgrund des Wasserbedarfs bei der Filtration und Wäsche des Nitro-AS-Mg-Salzes und des erforderlichen Recyclings der Waschwässer ist für die Kristallisation eine Mindestverdünnung der Schwefelsäure auf 50% und eine Temperatur zwischen 90 und 120°C vorteilhaft. Die Schwefelsäurekonzentration kann jedoch zwischen 40 und 60% variieren.

Als magnesiumhaltige Fällungsmittel kommen Magnesiumsalze wie Magnesiumsulfat oder Magnesiumcarbonat und Magnesiumoxid in Frage. Bevorzugt ist jedoch Magnesiumsulfat in Form von Bittersalz (Magnesiumsulfatheptahydrat). Diese Verbindungen können z. B. gelöst in Wasser oder im Gemisch mit Wasser angewendet werden.

Das Magnesiumsalz (Bittersalzlösung) wird in Mengen von 110 bis 130 Mol-%, bezogen auf den Gehalt an Nitro-Armstrong-Säure, eingesetzt. Optimal sind 120 bis 122 Mol-%.

Die vorteilhafteste Kristallisationstemperatur liegt zwischen 95 und 100°C.

Bevorzugt werden die Reaktionsstufen c) und d) wie folgt durchgeführt:

In einem Rührwerksbehälter wird das zur Verdünnung der Schwefelsäure notwendige Wasser vorgelegt. Die Nitrierungslösung der 1,5-Naphthalindisulfonsäure wird dann mit so einer Geschwindigkeit eingetragen, daß unter nur gelindem Kühlen die Temperatur der Lösung bei ca.

100°C bleibt. Danach erfolgt die Kristallisation des Nitro-AS-Mg-Salzes durch Zugabe einer Magnesiumionen abgebenden Verbindung, vorzugsweise einer wäßrigen Bittersalzlösung. Vorzugsweise werden dabei 1,20 bis 1,22 Mol Bittersalzlösung pro Mol Nitronaphthalinsulfonsäure eingesetzt und die Gesamtmenge innerhalb ca. 60 Minuten zudosiert. Die Konzentration beträgt in der wäßrigen Lösung in der Regel ca. 20 bis 25% $MgSO_4$. Nach der Kristallisation wird vorzugsweise noch ca. 60 Minuten bei ca. 90 bis 100°C nachgerührt, während ca. 60 Minuten auf ca. 20 bis 70°C, vorzugsweise 50 bis 60°C, abgekühlt und filtriert. Das Nutschgut wird mit Wasser nachgewaschen.

Um die geringe Menge an Restschwefelsäure aus dem feuchten Magnesiumsalz der Nitro-AS zu entfernen, genügt gewünschtenfalls eine Wäsche mit bis 3 kg Wasser pro Mol des Magnesiumsalzes. Diese Wäsche läßt sich im Gegensatz zu den bisher bekannten Verfahren technisch einfach und leicht durchführen.

Der so erhaltene Filterkuchen kann direkt in einer nachfolgenden Reduktionsstufe verarbeitet werden.

Eine kontinuierliche Kristallisationsvariante kann z. B. auch so gestaltet werden, daß man die beiden Lösungen von Nitro-AS-Reaktionsgemisch und Bittersalzlösung kontinuierlich ineinander düst, wobei die damit erfolgte synchrone Dosierung — gegebenenfalls in Gegenwart von Impfkeimen — optimales Fällungsergebnis zeigt.

Vorteile der Stufe c) sind, daß sich das gemäß dem neuen Kristallisationsverfahren erhaltene NAS-Mg-Salz mit einer Korngrößenverteilung von 30 bis 150 µ leicht filtrieren und waschen läßt, wodurch nur sehr wenig Waschflüssigkeit anfällt. Die Reinheit des Produktes steigt ganz erheblich. Die verunreinigenden isomeren Nebenprodukte konnten auf unter 2% gesenkt werden. Mußte der Filterkuchen gemäß bisherigen Verfahren mit 45%iger Schwefelsäure gewaschen werden, da die angefallenen Feinkristalle nur eine Korngröße zwischen 5 und 15 µ aufwiesen, so kann nach dem neuen Verfahren hergestelltes NAS-Mg-Salz mit Wasser gewaschen werden. Bisher mußte mit Filterpressen, nun kann mit Normalfiltern gearbeitet werden.

Im US-Patent 2 191 820 ist angegeben, daß die Nitrierung unter wasserfreien Bedingungen durchzuführen ist, um die Bildung von 1-Nitronaphthalin-4,8-disulfonsäure zu unterdrücken. Da Naphthalin-1,5-disulfonsäure als Tetrahydrat kristallisiert (s. Ullmann's Enzyklopädie der technischen Chemie, dritte Auflage, 12. Band, Seite 595) müssen dem Verfahren gemäß dem US-Patent 2 191 820 erhebliche Mengen Oleum zugeführt werden, um durch Bindung des Kristallwassers die Nitrierung in wasserfreiem Medium zu ermöglichen. Im erfindungsgemäßen Verfahren wird Naphthalin-1,5-disulfonsäure in Form des wasserfreien AS-AN eingesetzt und deshalb wesentlich weniger Oleum bzw. $SO_3$ benötigt, um das Nitriergemisch wasserfrei zu halten.

Es ist als ausgesprochen überraschend anzusehen, daß es unter den erfindungsgemäßen Bedingungen gelingt, das Magnesiumsalz der Nitro-AS in einer Form abzuscheiden, die gut filtrierbar und waschbar ist und die eine ausgezeichnete Abtrennung der Isomeren gestattet. Aus dem US-Patent 1 756 537 (1930) ist zwar bekannt, daß man aus Nitrergemischen, die bei der Nitrierung von Naphthalin-1,5-disulfonsäure oder deren Mischungen mit Naphthalin-1,6-disulfonsäure anfallen, durch Zugabe von Magnesiumsalz bei Temperaturen zwischen 0 und 100°C und Kristallisation bei 0 bis 10°C das Magnesiumsalz der Nitro-AS isolieren kann. In dem nachveröffentlichten SU-Erfinderschein 154 261 (1963) wird jedoch beschrieben, daß entgegen des in vorliegender Erfindung gefundenen Lösung das Magnesiumsalz der Nitro-AS in Form kleiner Kristalle anfällt, die schlecht filtrierbar und waschbar sind und aus denen die Abtrennung von Isomeren erschwert ist. Im SU-Erfinderschein 154 261 wird deshalb vogeschlagen, die Abtrennung von Nitro-AS aus Nitriergemischen in Form des Ammoniumsalzes vorzunehmen, weil dabei grobe, leicht filtrierbare und waschbare Kristalle anfallen.

Die mit dem erfindungsgemäßen Verfahren erzielbaren Ausbeuten sind hoch. Bezogen auf in die Sulfierung eingesetztes Naphthalin kann eine Ausbeute an isoliertem Magnesiumsalz der Nitro-AS von ca. 70% erhalten werden. Durch die konzentrierte Arbeitsweise ist auch die Raum/Zeit-Ausbeute sehr günstig. Die Reinheit des erfindungsgemäß hergestellten Magnesiumsalzes der Nitro-AS ist hoch, obwohl keine reine Naphthalin-1,5-disulfonsäure in die Nitrierung eingesetzt wird und bekanntermaßen bei der Nitrierung eine Reihe von unerwünschten Nebenprodukten entstehen (s. US-Patent 1 756 537). Der Verbrauch von Hilfsstoffen, wie Schwefelsäure und Oleum, ist gegenüber bekannten Verfahren stark vermindert. Es fallen deshalb auch nach der Isolierung des Magnesiumsalzes der Nitro-As wesentlich geringere Mengen verdünnter Säuren an. Ein weiterer Vorteil besteht darin, daß nach dem erfindungsgemäßen Verfahren eine praktisch von Schwefelsäure freie und salzfreie Nitro-AS erhalten werden kann und damit bei deren Weiterverarbeitung zu C-Säure auch salzarme C-Säure.

3-Nitronaphthalin-1,5-disulfonsäure bzw. das Magnesiumsalz davon ist eine wichtige Zwischenverbindung zur Herstellung der entsprechenden 2-Naphthylamin-4,8-disulfonsäure (C-Säure). C-Säure wird als Diazotierungskomponente für Baumwollfarbstoffe und als Zwischenprodukt zur Herstellung von 2-Naphthol-4,6-disulfonsäure verwendet (s. Ullmann's Enzyklopädie der technischen Chemie, 3. Auflage, 12. Band, Seite 629).

Die nachfolgenden Beispiele veranschaulichen das neue Verfahren.

## Beispiel 1

### Stufe a)

192,3 g Naphthalin (1,5 Mol) werden in 961,0 g Dichlormethan und gleichzeitig 360,3 g SO₃-flüssig in 1540 g Dichlormethan gelöst. Die beiden Lösungen werden auf −10°C gekühlt und in vorgelegtes, ebenfalls auf −10°C gekühltes Dichlormethan (600 g) gleichzeitig so zulaufen gelassen, daß die beiden Mischungen in gleichem Zeitintervall zudosiert sind. Das Reaktionsgemisch wird durch Verdampfungskühlung bei 0,093 bis 0,106 bar bei einer Temperatur zwischen −5 und −10°C gehalten. Das Destillat aus der Verdampfungskühlung, total 1209 g, wird zum Lösen von SO₃ wiederverwendet. Die Sulfonierungsreaktion ist nach ca. 1 bis 2 Stunden beendet. Man erhält eine Suspension des Sulfonierungsreaktionsgemisches, das formal betrachtet folgende Zusammensetzung hat:

55,6 Gew.-% Naphthalin-1,5-disulfonsäure,
0,2 Gew.-% Naphthalin-1,3-disulfonsäure,
5,9 Gew.-% Naphthalin-1,6-disulfonsäure,
2,3 Gew.-% Naphthalin-1,7-disulfonsäure,
2,7 Gew.-% Naphthalin-1,3,5-trisulfonsäure,
5,9 Gew.-% Naphthalin-1,3,6-trisulfonsäure,
0,8 Gew.-% Naphthalin-1,3,7-trisulfonsäure,
26,0 Gew.-% SO₃.

### Stufe b)

In einem separaten Kolben werden nun 800 g rohes Nitrierungsreaktionsgemisch (Rückführung aus der Stufe b) eines vorhergehenden Nitrieransatzes) und 732 g H₂SO₄ 100% vorgelegt und vermischt. Zur Suspension aus der Sulfonierungsreaktion (1,17 Mol Naphthalin-1,5-disulfonsäure und 0,33 Mol isomerer Di- und Trisulfonsäure) wird nun bei 20°C das Gemisch aus dem separaten Kolben zugegeben und langsam gerührt, bis sich das Dichlormethan in der oberen Schicht klar abtrennt, wonach es abgesaugt (dekantiert) werden kann. Das dadurch erhaltene Dichlormethan — ca. 1400 g — wird ohne Aufarbeitung in die Sulfonierungsreaktion recycliert. Die restlichen 414 g Dichlormethan werden bei 10 bis 30°C und 0,027 bar durch eine sogenannte Flash-Verdampfung abdestilliert und können ebenfalls wiederverwendet werden. Bei 0,027 bar wird die Temperatur des Destillationsrückstandes auf 20°C gehalten und bei dieser Temperatur in die nachfolgende Nitrierung eingespeist.

Die Nitrierung erfolgt durch simultane Dosierung von 312 g Mischsäure der Zusammensetzung:

33,1 Gew.-% HNO₃,
26,9 Gew.-% H₂SO₄,
40,0 Gew.-% SO₃

und 2083 g Destillationsrückstandsbrei bei 30 bis 40°C in 2400 g Restmenge des Nitriergemisches aus der Stufe b) eines vorausgegangenen Ansatzes. Danach läßt man unter ständigem Rühren bei 40°C ausreagieren, was insgesamt ca. 4 Stunden in Anspruch nimmt.

Ein Teil = 800 g (ca. 1/6) der erhaltenen rohen Reaktionsmischung wird in die vorher beschriebene Zweiphasentrennung rückgeführt.

### Stufe c)

Ein zweiter Teil (ca. 2/6) wird folgendermaßen zum Produkt aufgearbeitet: Im korrosionsfesten Reaktor mit Doppelmantel werden 1123 g kaltes Wasser vorgelegt und innerhalb von 30 Minuten 1594 g des Nitriergemisches enthaltend 336 g NAS (1 Mol) unter starkem Rühren zugegeben. Durch leichtes Kühlen wird die Temperatur auf 100°C gehalten. Das Reaktionsgemisch geht dabei vollständig in Lösung. Nach beendetem Zulauf erfolgt die selektive Fällung des Nitro-Armstrongsäure-Mg-Salzes durch Zudosieren von 654 g einer wäßrigen Bittersalzlösung mit einem MgSO₄-Gehalt von 158 g (1,3 Mol) während 60 Minuten.

### Stufe d)

Danach werden während 5 Minuten 390 g Waschlauge aus einer vorhergehenden Fällung zugegeben. Die Suspension wird nun noch 60 Minuten bei 95°C langsam gerührt, auf 60°C abgekühlt und weitere 60 Minuten gerührt. Nun erfolgt die Filtration der grobkristallinen Suspension. Der Filterkuchen wird danach abgepreßt und die Mutterlauge abgesaugt, wonach mit 300 g kaltem Wasser gewaschen und trockengesaugt wird. Mutter- und Waschlauge werden separat aufgefangen, da nur die Waschlauge recycliert wird.

Es werden ca. 590 g feuchtes 3-Nitronaphthalin-1,5-disulfonsäuredimagnesiumsalz erhalten, was 366 g Trockensubstanz 100%ig entspricht (ca. 70% Ausbeute, bezogen auf eingesetztes Naphthalin). Der Anteil an isomeren Nebenprodukten im Produkt ist kleiner als 1,5%.

Ein dritter Teil, d. h. die Restmenge der rohen Nitrierungsreaktionsmischung (2400 g, ca. 3/6) dient, insbesondere bei Durchführung des Verfahrens in technischem Maßstab, wieder als Vorlage für einen weiteren nachfolgenden Nitrierungsansatz.

Wird bei der Nitrierungsreaktion die Mischsäure und der Destillationsrückstand nicht simultan sondern taktweise hintereinander so zugegeben, daß man jeweils kleine Anteile AS-AN-Brei vorlegt und die erforderliche Menge Mischsäure nachdosiert, so erhält man das Endprodukt in gleicher Ausbeute und Qualität.

## Beispiel 2

Es wurde wie in Beispiel 1 verfahren, wobei aus dem Sulfonierungsprodukt von Naphthalin das Dichlormethan jedoch nicht entfernt, sondern dieses in Form einer 30gew.-%igen Suspension in Dichlormethan in der weiteren Nitrierungsreaktion eingesetzt wurde. Das ergab eine Ausbeute des Magnesiumsalzes von 3-Nitronaphthalin-1,5-disulfonsäure von 67% (bezogen auf Naphthalin) bzw. von 87% (bezogen auf Naphthalin-1,5-disulfonsäure) in Form des eingesetzten Sulfonierungsproduktes. Das Dichlormethan wurde während der Zugabe des Nitriergemisches langsam destillativ entfernt.

## Patentansprüche

1. Verfahren zur Herstellung des Magnesiumsalzes von 3-Nitronaphthalin-1,5-disulfonsäure durch Sulfonierung von Naphthalin, Nitrierung des Sulfonierungsproduktes und Abtrennung des reinen Magnesiumsalzes von 3-Nitronaphthalin-1,5-disulfonsäure, dadurch gekennzeichnet, daß man

a) Naphthalin mit flüssigem $SO_3$ in Gegenwart eines reaktionsinerten organischen Lösungsmittels bei Temperaturen im Bereich −40 bis +20°C sulfoniert, wobei das Verhältnis von zugegebenem $SO_3$ zu zugegebenem Naphthalin während der gesamten Zugabe im Bereich von 2,5 bis 5,0 Mol $SO_3$ pro Mol Naphthalin liegt,

b) dem erhaltenen Reaktionsgemisch eine Mischung, bestehend aus in etwa gleichen Teilen 100%iger Schwefelsäure und ausreagierter roher Nitriermasse aus der Stufe b) zusetzt, wodurch ein Zweiphasen-System resultiert, von dem die obere organische Lösungsmittel-Phase dekantiert und der Rest des verbliebenen organischen Lösungsmittels aus dem Armstrongsäure-Anhydrid-Säurebrei destillativ, vorteilhaft durch Flashverdampfung oder gewünschtenfalls bei der nachfolgenden Nitrierung zur gleichzeitigen Abführung der Nitrierungswärme, oder erst nach erfolgter Nitrierung entfernt wird, wonach man zum Rückstand die zur Nitrierung erforderliche Salpetersäuremenge zweckmäßig im Gemisch mit Schwefelsäure und Schwefeltrioxid bei einer Temperatur zwischen 10 und 60°C versetzt und bei dieser Temperatur während 1 bis 6 Stunden nitriert,

c) danach zunächst Wasser und dann in Abwesenheit des inerten organischen Lösungsmittels bei 90 bis 120°C so viel einer Magnesiumionen abgebenden Verbindung zugibt, daß pro Mol 3-Nitronaphthalin-1,5-disulfonsäure 1,1 bis 1,3 Mol Magnesiumionen zugegeben werden und eine Schwefelsäurekonzentration im Bereich von 40 bis 60 Gew.-% (bezogen auf die Schwefelsäure und Wasser) eingehalten wird, und

d) das auskristallisierte Magnesiumsalz der 3-Nitronaphthalin-1,5-disulfonsäure bei einer Temperatur im Bereich von 20 bis 70°C abtrennt und anschließend gegebenenfalls mit maximal 60gew.-%iger Schwefelsäure und/oder Wasser wäscht.

2. Sulfonierungsverfahren gemäß Anspruch 1, dadurch gekennzeichnet, daß man $SO_3$ und Naphthalin, jeweils getrennt in einem inerten organischen Lösungsmittel, vorzugsweise in einem aliphatischen Chlorkohlenwasserstoff mit 1 bis 3 Kohlenstoffatomen, insbesondere Dichlormethan, löst und beide Lösungen bei ca. −10 bis −5°C vereinigt, wobei während der gesamten Zugabe ein Molverhältnis von zugegebenem $SO_3$ zu zugegebenem Naphthalin im Bereich von 2,5 bis 3,6, vorzugsweise im Bereich von 2,8 bis 3,2, eingehalten wird.

3. Ausführungsform des Verfahrens gemäß Anspruch 1, dadurch gekennzeichnet, daß man den nach der Phasentrennung erhaltenen Armstrongsäure-Anhydrid-Brei und Salpetersäure simultan oder taktweise hintereinander in ein vorgelegtes rohes Nitriergemisch der Stufe b) dosiert und anschließend nitriert.

4. Ausführungsform des Verfahrens gemäß Anspruch 1, dadurch gekennzeichnet, daß man das rohe Nitriergemisch der Stufe b) in 3 Teile aufteilt, wovon ein Teil zum Produkt 3-Nitronaphthalin-1,5-disul-

7

fonsäure aufgearbeitet wird, der zweite Teil mit 100%iger Schwefelsäure im Verhältnis 1 : 1 gemischt wird und diesen zum Zwecke der Phasentrennung dem Sulfonierungsgemisch zugibt und der dritte Teil als Vorlage einer nächsten Nitrierungsreaktion verwendet.

5. Verfahren nach Ansprüchen 1 bis 4, dadurch gekennzeichnet, daß man das Lösungsmittel nach der Sulfonierungsstufe vollständig entfernt.

6. Verfahren nach Ansprüchen 1 bis 4, dadurch gekennzeichnet, daß man die restlichen nach der Dekantation noch vorhandenen Lösungsmittelanteile erst während der Nitrierungsreaktionsstufe entfernt.

7. Verfahren nach Ansprüchen 1 bis 4, dadurch gekennzeichnet, daß das zur Phasentrennung nach der Sulfonierungsreaktion einzusetzende Säuregemisch aus konzentrierter Schwefelsäure und ausreagierter roher Nitriermasse aus mindestens 6 Gewichtsteilen, und die beiden einzelnen Komponenten aus mindestens je 3 Gewichtsteilen pro 1 Teil in die Sulfonierungsreaktion eingesetztem Naphthalin bestehen.

8. Verfahren nach Ansprüchen 1 bis 7, dadurch gekennzeichnet, daß zur Nitrierung 1 bis 1,5 Mol Salpetersäure in Form von Mischsäure pro Mol Naphthalin verwendet wird.

9. Verfahren nach Ansprüchen 1 bis 8, dadurch gekennzeichnet, daß die zur Nitrierung verwendete Mischsäure neben Schwefelsäure 0,8 bis 1,2 Mol $SO_3$ pro Mol Salpetersäure enthält.

10. Verfahren gemäß Anspruch 1, dadurch gekennzeichnet, daß man die Phasentrennung Lösungsmittel-Säure kontinuierlich durchführt.

11. Verfahren nach Anspruch 1, dadurch gekennzeichnet, daß man die als Produkt entstandene 3-Nitronaphthalin-1,5-disulfonsäure nach vorherigem Lösen des Reaktionsgemisches in Wasser bei 90 bis 120° C als Magnesiumsalz auskristallisiert.

12. Verfahren nach Anspruch 11, dadurch gekennzeichnet, daß man die Magnesiumsalzkristallisation mittels Magnesiumsalzlösungen oder -suspensionen bei einer Temperatur zwischen 95 und 100° C durchführt, und Magnesiumsalz in Mengen verwendet, daß die Magnesiumionen in Mengen von 1,1 bis 1,3 Mol pro Mol Nitro-Armstrongsäure vorhanden sind.

13. Verfahren nach Anspruch 1, dadurch gekennzeichnet, daß man das abfiltrierte Magnesiumsalz der Nitro-Armstrongsäure nur mit Wasser ohne vorherige Schwefelsäurewäsche wäscht.

14. Verfahren gemäß Anspruch 1, dadurch gekennzeichnet, daß man zur Magnesiumsalzfällung Magnesiumsulfat verwendet.

## Claims

1. A process for producing the magnesium salt of 3-nitronaphthalene-1,5-disulfonic acid by sulfonating naphthalene, nitrating the sulfonation product, and separating the pure magnesium salt of 3-nitronaphthalene-1,5-disulfonic acid, which process comprises

a) sulfonating naphthalene with liquid $SO_3$ in the presence of an inert organic solvent at temperatures between −40 and +20° C, the ratio of added $SO_3$ to added naphthalene being during the whole course of addition in the range of 2.5 to 5.0 mols of $SO_3$ per mol of naphthalene;

b) adding to the reaction mixture obtained a mixture consisting of about equal parts of 100% sulfuric acid and fully reacted crude nitration mixture from Stage b), separating from the resulting two-phase system the upper organic solvent phase by decanting, and removing from the Armstrong's acid anhydride slurry the remainder of organic solvent by distillation, advantageously by flash evaporation, or, if desired, allowing it to be drawn off during subsequent nitration with simultaneous elimination of the heat of nitration, or extracting it after nitration has been performed, subsequently adding to the residue the amount of nitric acid required for nitration, advantageously in admixture with sulfuric acid and sulfur trioxide, at a temperature of between 10 and 60° C, and nitrating at this temperature for 1 to 6 hours;

c) adding then firstly water and afterwards, in the absence of the inert organic solvent, at 90 to 120° C, a compound releasing magnesium ions, the amount being such that, per mol of 3-nitronaphthalene-1,5-disulfonic acid, 1.1 to 1.3 mols of magnesium ions are added, and a sulfuric acid concentration of between 40 and 60 per cent by weight (relative to the sulfuric acid and water) is maintained; and

d) separating the crystallised magnesium salt of 3-nitronaphthalene-1,5-disulfonic acid at a temperature of between 20 and 70° C, and subsequently optionally washing it with a maximum of 60 per cent by weight of sulfuric acid and/or water.

2. A sulfonation process according to Claim 1, wherein $SO_3$ and naphthalene are each dissolved separately in an inert organic solvent, preferably in an aliphatic chlorinated hydrocarbon having 1 to 3 carbon atoms, especially dichloromethane, and te two solutions are then combined at about −10 to −5° C, a molar ration of added $SO_3$ to added naphthalene in the range of 2.5 to 3.6, preferably 2.8 to 3.2, being maintained during the whole course of the addition.

3. Modification of the process according to Claim 1, wherein the Armstrong's acid anhydride slurry

8

obtained after phase separation and nitric acid are added, either simultaneously or one after the other, to a crude nitration mixture, and the mixture is subsequently nitrated.

4. Modification of the process according to Claim 1, wherein the crude nitration mixture is divided into three portions, of which one portion is processed to give 3-nitronaphthalene-1,5-disulfonic acid; the second portion is mixed with 100% sulfuric acid in the ratio of 1 : 1 and the mixture is added to the sulfonation mixture for the purpose of phase separation; and the third portion is used as material for a subsequent nitration reaction.

5. A process according to Claims 1 to 4 inclusive, wherein the solvent is completely removed after the sulfonation stage.

6. A process according to Claim 1 to 4 inclusive, wherein the residual solvent present after decantation is not removed until during the nitration reaction stage.

7. A process according to Claims 1 to 4 inclusive, wherein the acid mixture of concentrated sulfuric acid and fully reacted crude nitration mixture, which mixture is to be used for phase separation after the sulfonation reaction, consists of at least 6 parts by weight, and each of the two individual components of at least 3 parts by weight, per 1 part of naphthalene used in the sulfonation reaction.

8. A process according to Claims 1 to 7 inclusive, wherein there are used for nitration 1 to 1.5 mols of nitric acid, in the form of mixed acid, per mol of naphthalene.

9. A process according to Claims 1 to 8 inclusive, wherein the mixed acid used for nitration contains, besides sulfuric acid, 0.8 to 1.2 mols of $SO_3$ per mol of nitric acid.

10. A process according to Claim 1, wherein phase separation of solvent and acid is performed continuously.

11. A process according to Claim 1, wherein the 3-nitronaphthalene-1,5-disulfonic acid formed as product crystallises out as magnesium salt after previous dissolving of the reaction mixture in water at 90 to 120°C.

12. A process according to Claim 11, wherein the crystallisation of magnesium salt is performed by means of magnesium salt solutions or suspensions at a temperature of between 95 and 100°C, and magnesium salt is used in such amounts, that the magnesium ions are present in amount of 1.1 to 1.3 mols per mol of nitro-Armstrong's acid.

13. A process according to Claim 1, wherein the filtered-off magnesium salt of nitro-Armstrong's acid is washed only with water without previous washing with sulfuric acid.

14. A process according to Claim 1, wherein magnesium sulfate is used for the precipitation of magnesium salt.

## Revendications

· 1. Procédé pour la préparation du sel de magnésium de l'acide 3-nitronaphtalène-1,5-disulfonique par sulfonation du naphtalène, nitration du produit de sulfonation et séparation du sel de magnésium pur de l'acide 3-nitronaphtalène-1,5-disulfonique, caractérisé par le fait que

a) on sulfone le naphtalène avec du $SO_3$ liquide en présence d'un solvant organique inerte vis-à-vis de la réaction, à des températures allant de —40 à +20°C, le rapport du $SO_3$ ajouté au naphtalène ajouté pendant l'addition globale se trouvant dans la gamme de 2,5 à 5 moles de $SO_3$ par mole de naphtalène,

b) au mélange réactionnel obtenu, on ajoute un mélange constitué par des parties sensiblement égales d'acide sulfurique à 100% et de masse nitrée brute ayant cessé de réagir provenant du stade b), ce qui donne un système à deux phases, duquel la phase solvant organique supérieur décante et le reste du solvant organique résiduaire est éliminé par distillation de la bouillie acide, acide d'Armstrong-anhydride, avantageusement par évaporation flash ou, si on le désire, au cours de la nitration ultérieure afin d'enlever simultanément la chaleur de nitration, ou bien seulement une fois la nitration effectuée, après quoi on ajoute au résidu la quantité d'acide nitrique nécessaire à la nitration, de préférence en mélange avec l'acide sulfurique et le trioxyde de soufre, à une température comprise entre 10 et 60°C, et on nitre à cette température pendant 1 à 6 heures,

c) ensuite, on ajoute tout d'abord de l'eau puis, en l'absence de solvant organique inerte à 90—120°C, une quantité suffisante d'un composé donnant des ions magnésium pour que par mole d'acide 3-nitronaphtalène-1,5-disulfonique soit ajouté 1,1 à 1,3 mole d'ions magnésium et que soit maintenue une concentration en acide sulfurique allant de 40 à 60% en poids (rapporté à l'acide sulfurique et l'eau), et

d) on sépare le sel de magnésium cristallisé de l'acide 3-nitronaphtalène-1,5-disulfonique à une température allant de 20 à 70°C, puis on le lave éventuellement avec de l'acide sulfurique à 60% maximum et/ou de l'eau.

2. Procédé de sulfonation selon la revendication 1, caractérisé par le fait qu'on dissout le $SO_3$ et le naphtalène à chaque fois séparément dans un solvant organique inerte, de préférence dans un hydrocarbure chloré aliphatique ayant 1 à 3 atomes de carbone, en particulier dans le dichlorométhane, et qu'on réunit les deux solutions à environ −10 à −5°C, tout en maintenant, pendant l'addition globale, un rapport molaire du $SO_3$ ajouté au naphtalène ajouté dans la gamme de 2,5 à 3,6, de préférence dans la gamme de 2,8 à 3,2.

3. Forme réalisation du procédé selon la revendication 1, caractérisée par le fait qu'on dose la bouillie acide Armstrong-anhydride, obtenue après la séparation de phases, et l'acide nitrique, simultanément ou successivement d'une façon rythmique dans un mélange nitré brut placé au préalable puis on nitre.

4. Forme de réalisation du procédé selon la revendication 1, caractérisée par le fait qu'on divise le mélange nitré bruten trois parties, à partir desquelles une partie est transformée en le produit acide 3-nitronaphtalène-1,5-disulfonique, la deuxième partie est mélangée avec de l'acide sulfurique à 100% dans un rapport 1 : 1 et ce mélange est ajouté au mélange de sulfonation en vue de la séparation de phases, et la troisième partie est utilisée comme produit de base d'une réaction ultérieure de nitration.

5. Procédé selon les revendications 1 à 4, caractérisé par le fait qu'on enlève complètement le solvant après le stade de sulfonation.

6. Procédé selon les revendications 1 à 4 caractérisé par le fait qu'on n'enlève les quantités de solvant encore présentes restant après la décantation que pendant le stade de la réaction de nitration.

7. Procédé selon les revendications 1 à 4, caractérisé par le fait que le mélange d'acides à mettre en oeuvre pour la séparation de phase après la réaction de sulfonation est constitué par de l'acide sulfurique concentré et par de la masse nitrée brute ayant cessé de réagir à raison d'au moins 6 parties en poids, et que chacun des deux constituants consiste en au moins trois parties en poids, pour une partie du naphtalène mis en oeuvre dans la réaction de sulfonation.

8. Procédé selon les revendications 1 à 7, caractérisé par le fait que pour la nitration, 1 à 1,5 mole d'acide nitrique est utilisé sous forme de mélange sulfonitrique par mole de naphtalène.

9. Procédé selon les revendications 1 à 8, caractérisé par le fait que le mélange sulfonitrique utilisé pour la nitration contient en plus de l'acide sulfurique 0,8 à 1,2 mole de $SO_3$ par mole d'acide nitrique.

10. Procédé selon la revendication 1, caractérisé par le fait qu'on effectue en continue la séparation de phases solvant-acide.

11. Procédé selon la revendication 1, caractérisé par le fait qu'on sépare par cristallisation l'acide 3-nitronaphtalène-1,5-disulfonique formé comme produit après dissolution préalable du mélange réactionnel dans l'eau, à 90 à 120° C sous forme de sel de magnésium.

12. Procédé selon la revendication 11, caractérisé par le fait qu'on effectue la cristallisation du sel de magnésium au moyen de solutions ou de suspensions de sel de magnésium, à une température comprise entre 95 et 100°C, et qu'on utilise le sel de magnésium en une quantité suffisante pour les ions magnésium soient présents à raison de 1,1 à 1,3 mole par mole d'acide nitro-Armstrong.

13. Procédé selon la revendications 1, caractérisé par le fait qu'on lave le sel de magnésium de l'acide nitro-Armstrong séparé par filtration seulement avec de l'eau sans lavage préalable avec l'acide sulfurique.

14. Procédé selon la revendication 1, caractérisé par le fait qu'on utilise le sulfate de magnésium pour la précipitation du sel de magnésium.